(19) Europäisches Patentamt
European Patent Office
Office européen des brevets

(11) Veröffentlichungsnummer: **0 325 144**
**A2**

(12) **EUROPÄISCHE PATENTANMELDUNG**

(21) Anmeldenummer: 89100334.5

(22) Anmeldetag: 10.01.89

(51) Int. Cl.⁴: **C07C 31/125** , **C07C 29/04**

(30) Priorität: 21.01.88 DE 3801578

(43) Veröffentlichungstag der Anmeldung:
26.07.89 Patentblatt 89/30

(84) Benannte Vertragsstaaten:
**DE FR IT NL**

(71) Anmelder: **EC ERDÖLCHEMIE GMBH**
**Postfach 75 20 02**
**D-5000 Köln 71(DE)**

(72) Erfinder: **Malessa, Reiner, Dr.**
**Naukler Strasse 57**
**D-7400 Tübingen(DE)**
Erfinder: **Schleppinghoff, Bernard, Dr.**
**Adolf-Kolping-Strasse 5**
**D-4047 Dormagen(DE)**

(74) Vertreter: **Gerhards, Peter, Dr. et al**
**Bayer AG Konzernverwaltung RP**
**Patentabteilung**
**D-5090 Leverkusen 1 Bayerwerk(DE)**

(54) **Verfahren zur Herstellung von tert.-Amylalkohol (TAA).**

(57) Bei der Herstellung von tert.-Amylalkohol (TAA) durch Hydratisierung von i-Amylenen an sauren Kationenaustauschern können diese Kationenaustauscher zur Erhöhung des TAA-Gehalts im Produktgemisch und zur Verminderung der Oligomerisierung der i-Amylene mit 0,02 - 0,3 Mol eines amphoteren Elements je 1 kg trockenen Kationenaustauschers beladen werden.

EP 0 325 144 A2

## Verfahren zur Herstellung von tert.-Amylalkohol (TAA)

Die Erfindung betrifft ein Verfahren zur Herstellung von tert.-Amylalkohol (TAA) durch Hydratation von i-Amylenen an einem sauren Kationenaustauscher.

Aus US 2 813 908 ist es bereits bekannt, Olefine an Sulfonsäuregruppen enthaltenden Styrol-Divinylbenzol-Polymeren bei 121 - 218° C und 200 - 106 bar zu hydratisieren. Dieses Verfahren führt bei Verwendung von i-Buten zu einer starken Bildung von i-Buten-Oligomeren.

Aus US 3 257 469 ist weiterhin die Herstellung von TAA an sauren Kationenaustauschern durch Umsetzung von i-Amylen mit einem molaren Überschuß an Wasser bekannt. Hier wird bei 38 - 204° C und Drücken bis zu 106 bar gearbeitet; zur Erhöhung des Umsatzes werden Lösungsmittel verwendet, von denen i-Propanol und Aceton besonders herausgestellt werden. Dieses Verfahren umfaßt damit notwendigerweise die Abtrennung des mitverwendeten Lösungsmittels aus dem Reaktionsgemisch. In diesem Verfahren beträgt die Menge des Acetons 60 - 75 % des gesamten Einsatzstroms (US 4 182 920).

Aus allen beschriebenen Verfahren zur Hydratation von i-Olefinen ist dem Fachmann bekannt, daß beim Einsatz von i-Buten hohe Umsätze erreicht werden, daß aber beim Einsatz von i-Amylenen wegen der deutlich ungünstigeren Gleichgewichtslage die Umsätze häufig unbefriedigend sind. Die gleichzeitige Bildung von i-Olefin-Oligomeren ist eine unerwünschte Nebenreaktion bei allen Hydratationsverfahren.

Es wurde nun überraschend gefunden, daß kleine Beladungsmengen amphoterer Elemente auf den verwendeten sauren Kationenaustauschern den Umsatz bei der TAA-Herstellung deutlich erhöhen und gleichzeitig die Oligomerenbildung unterdrücken.

Es wurde ein Verfahren zur Herstellung von tert.-Amylalkohol (TAA) durch Hydratation von i-Amylenen an sauren Kationenaustauschern gefunden, das dadurch gekennzeichnet ist, daß der Kationenaustauscher pro kg Trockenmasse mit 0,02 - 0,3 Mol eines amphoteren Elements beladen wird.

In bevorzugter Weise wird jedes kg trockener Kationenaustauscher mit 0,03 - 0,2 Mol, besonders bevorzugt mit 0,04 - 0,15 Mol eines amphoteren Elements beladen.

Als amphotere Elemente sind dem Fachmann solche bekannt, die sowohl Kationen als auch Anionen bilden können, wobei vielfach verschiedene Wertigkeitsstufen vorliegen können. Als solche amphoteren Elemente seien beispielsweise genannt: Aluminium, Chrom, Vanadin, Titan, Zirkon, Niob, Tantal, Arsen, Antimon, Molybdän, Wolfram, Mangan und Rhenium. In bevorzugter Weise seien genannt: Aluminium, Chrom, Vanadin, Titan, Zirkon, Molybdän und Wolfram. In besonders bevorzugter Weise seien genannt: Aluminium, Chrom und Vanadin. Für die erfindungsgemäße Beladung von sauren Kationenaustauschern werden solche Verbindungen eingesetzt, in denen die amphoteren Elemente in kationischer Form vorliegen.

Als saure Kationenaustauscher können alle bekannten Typen, wie sulfonierte Phenol-Formaldehyd-Harze, sulfonierte Cumaron-Inden-Kondensationsprodukte, sulfonierte Polystyrole, sulfonierte Styrol-Divinylbenzol-Harze usw. eingesetzt werden; sie werden erfindungsgemäß in ihrer $H^+$-Form eingesetzt. In bevorzugter Weise werden sulfonierte Styrol-Divinylbenzol-Harze mit einem Vernetzungsgrad (Gehalt an Divinylbenzol) von 2 - 65 %, bevorzugt 8 - 25 %, eingesetzt. Solche sauren Kationenaustauscher sind dem Fachmann bekannt und unter vielen Bezeichnungen im Handel.

Zur erfindungsgemäßen Beladung des sauren Kationenaustauschers wird beispielsweise wie folgt vorgegangen: Der saure Kationenaustauscher wird mit voll entsalztem oder destilliertem Wasser gewaschen und anschließend vorteilhaft getrocknet, um definierte Ausgangsmassen zu haben. Eine solche Trocknung kann etwa für 24 Stunden bei 90° C im Wasserstrahlvakuum vorgenommen werden. Nach dem Wägen wird der saure Kationenaustauscher in Wasser gequollen und in üblicher Weise durch Zugabe von Elementsalzlösungen beladen. Die Menge der Elementsalzlösung wird nach der gewünschten Beladung gewählt; ihre Konzentration beträgt beispielsweise 0,01-1 Mol/l, bevorzugt 0,01-0,5 Mol/l, typischerweise etwa 0,02 Mol/l Lösungsmittel (im allgemeinen Wasser). Die Bezeichnung kann in Massenprozent Elemention pro Masse des getrockneten Kationenaustauschers oder in Mol des Elements pro Masse getrocknetem Kationenaustauscher angegeben werden.

Der erfindungsgemäß einzusetzende beladene Kationenaustauscher kann absatzweise, beispielsweise in einem Rührautoklaven, zur Hydratation eingesetzt werden. Er kann aber auch als fest angeordnetes Katalysatorbett in einem Röhren- oder Kolonnenreaktor verwendet werden. Die Variante in einem Röhren- oder Kolonnenreaktor läßt sich kontinuierlich durchführen und ist daher für die technische Durchführung bevorzugt. Hierbei wird der beladene Kationenaustauscher mit einer Menge an gesamtem Einsatzmaterial beaufschlagt, die eine Raumgeschwindigkeit (LHSV = Liquid Hourly Space Velosity) von 0,05 - 1 l Reaktionsgemisch pro Liter Katalysator und Stunde bedeutet.

Das erfindungsgemäße Verfahren kann bei einer Temperatur von 20 - 100° C, bevorzugt 35 - 80° C, unter Normaldruck, vermindertem oder erhöhtem Druck, durchgeführt werden. Der Druck an sich ist für das erfindungsgemäße Verfahren unkritisch. So kann beispielsweise in der absatzweisen Fahrweise im Autoklaven unter dem sich automatisch einstellenden Eigendruck des Reaktionsgemisches oder unter zusätzlichem Inertgasdruck ($N_2$, $CO_2$, $CH_4$ u. a.) gearbeitet werden. Im Röhren- bzw. Kolonnenreaktor kann beispielsweise das Einsatzgemisch in flüssiger Phase über den beladenen Kationenaustauscher gefahren werden. Bei höherer Reaktionstemperatur wird hierzu der Druck so weit angehoben, daß die Reaktionstemperatur deutlich unterhalb der zugehörigen Siedetemperatur der i-Amylene liegt. Das Einsatzgemisch kann jedoch auch in der Rieselphase durchgeführt werden, wobei ein Teil des i-Amylens in die Dampfphase übertreten kann und am Kolonnenkopf bei totalem Rücklauf wieder auf den beladenen Kationenaustauscher geleitet wird. Bei einer solchen Variante in der Rieselphase wird man daher in der Nähe des Siedepunkts arbeiten. Auch hierzu kann ein geringfügig erhöhter Druck nötig sein, wenn die Reaktionstemperatur angehoben werden soll. Eine bevorzugte Ausführung dieser Rieselphasen-Variante ist jedoch die bei Normaldruck und etwa 35 - 40° C, also in der Nähe des Siedepunktes der i-Amylene.

Weitere Varianten des erfindungsgemäßen Verfahrens sind die Benutzung mehrerer Reaktoren, wobei sowohl die i-Amylene als auch das erforderliche Reaktionswasser auf die einzelnen Reaktoren aufgeteilt werden können.

Als i-Amylene kommen das 2-Methyl-buten-(1) und das 2-Methyl-buten-(2) in Frage. Sie können sowohl in reiner Form oder in Form von Destillationsschnitten, in denen sie vergesellschaftet mit anderen Kohlenwasserstoffen vorkommen, eingesetzt werden. Aufgrund der weiter oben bereits geschilderten Gleichgewichtslage zur Bildung des TAA ist es jedoch bevorzugt, die i-Amylene in angereicherter oder in im wesentlichen reiner Form einzusetzen, da beim Einsatz eines Destillationsschnittes das TAA in stark verdünnter Form erhalten würde.

Die Menge des eingesetzten Hydratationswassers ist grundsätzlich unkritisch und erlaubt demzufolge den Einsatz überstöchiometrischer, äquimolarer bzw. unterstöchiometrischer Mengen. In bevorzugter Weise wird jedoch etwa die stöchiometrische Menge an Hydratationswasser eingesetzt, die entsprechend der Gleichgewichtslage bei der eingestellten Reaktionstemperatur dem umzusetzenden i-Amylen entspricht. Diese Gleichgewichtslage kann der Fachmann durch wenige orientierende Versuche bestimmen.

Vergleichsbeispiel und Beispiele 1 - 4

Als Kationenaustauscher wurde ein sulfoniertes Styrol-Divinylbenzol-Harz (Verkaufsprodukt SPC 118 der BAYER AG) in der $H^+$- Form eingesetzt. Im Vergleichsbeispiel wurde keine Beladung vorgenommen. In den Beispielen 1 - 4 wurde die in der Tabelle angegebene Beladung mit Hilfe von Aluminiumsulfat-, Chrom(III)-chlorid- bzw. Vanadylsulfat-Lösung vorgenommen.

200 ml des Kontaktes wurden in einem Rührautoklaven mit 267 g i-Amylenen, 5 g TAA und 17 g Wasser beaufschlagt und bei 50° C 4 Stunden umgesetzt. Die Tabelle zeigt die Ergebnisse.

| Beispiel Nr. | Beladung (Massen-%) | TAA im Reaktionsprodukt (Massen-%) |
|---|---|---|
| Vergleich | - | 20 |
| 1 | 0,1 % $Al^{3+}$ | 25 |
| 2 | 0,3 % $Al^{3+}$ *) | 27 |
| 3 | 0,6 % $Cr^{3+}$ *) | 25 |
| 4 | 0,8 % $VO^{2+}$ *) | 27 |

*) Entsprechend etwa 0,01 Mol je 100 g getrocknetem SPC 118.

Beispiel 5

Die Kontakte aus dem Vergleichsbeispiel und dem Beispiel 2 wurden in einer Anordnung aus 2 Rohrreaktoren unter identischen Bedingungen untersucht. Hierzu stellte man bei einem einheitlichen Druck von 10 bar im ersten Reaktor eine Temperatur von 70° C und im zweiten Reaktor eine Temperatur von 60° C ein. Das Volumen des ersten Reaktors betrug 2,2 l, das Volumen das zweiten Reaktors 3 l. Es wurden pro Stunde 650 g i-Amylene und 40 g $H_2O$ eindosiert.

Folgende Ergebnisse wurden erhalten:

| Katalysator | TAA im Produkt (Massen-%) | Gehalt an Dimeren (Massen-%) |
|---|---|---|
| SPC 118 | 21 | 9 |
| SPC 118 + 0,3 % $Al^{3+}$ | 21 | <0,01 |

## Ansprüche

1. Verfahren zur Herstellung von tert.-Amylalkohol (TAA) durch Hydratation von i-Amylenen an sauren Kationenaustauschern, dadurch gekennzeichnet, daß der Kationenaustauscher pro kg Trockenmasse mit 0,02 -0,3 Mol eines amphoteren Elements beladen wird.

2. Verfahren nach Anspruch 1, dadurch gekennzeichnet, daß jedes kg trockener Kationenaustauscher mit 0,03 - 0,2 Mol eines amphoteren Elements beladen wird.

3. Verfahren nach Anspruch 2, dadurch gekennzeichnet, daß jedes kg trockener Kationenaustauscher mit 0,04 - 0,15 Mol eines amphoteren Elements beladen wird.

4. Verfahren nach Anspruch 1, dadurch gekennzeichnet, daß als amphotere Elemente Aluminium, Chrom, Vanadin, Titan, Zirkon, Niob, Tantal, Arsen, Antimon, Molybdän, Wolfram, Mangan oder Rhenium eingesetzt werden.

5. Verfahren nach Anspruch 4, dadurch gekennzeichnet, daß als amphotere Elemente Aluminium, Chrom, Vanadin, Titan, Zirkon, Molybdän oder Wolfram eingesetzt werden.

6. Verfahren nach Anspruch 5, dadurch gekennzeichnet, daß als amphotere Elemente Aluminium, Chrom oder Vanadin eingesetzt werden.

4